# EUROPEAN PATENT SPECIFICATION

(11) **EP 1 926 811 B1**
(45) Date of publication and mention of the grant of the patent: **07.03.2012**
(21) Application number: 06803596.3
(22) Date of filing: 14.09.2006
(51) Int. Cl.: C12N 1/20, C07K 14/31

(54) **PROTEIN A PRODUCTION AND PURIFICATION WITHOUT USING ANIMAL DERIVED COMPONENTS**
HERSTELLUNG UND REINIGUNG VON PROTEIN A OHNE DIE VERWENDUNG VON TIERISCHEN KOMPONENTEN
PRODUCTION ET PURIFICATION DE PROTEINE A SANS UTILISATION DE COMPOSANTS D'ORIGINE ANIMALE

(30) Priority: 20.09.2005 US 718759 P
(43) Date of publication of application: 04.06.2008
(73) Proprietor: Avantor Performance Materials, Inc., Center Valley PA 18034 (US)
(72) Inventor: DEORKAR, Nandu, Cedar Knolls, New Jersey 07927 (US); GUO, Wei, Bethlehem, Pennsylvania 18018 (US); MAGEE, Steve, Bethlehem, Pennsylvania 18017 (US)
(74) Representative: Cockerton, Bruce Roger
(86) International application number: PCT/US2006/035837
(87) International publication number: WO 2007/035410

(56) References cited:
- EP-A- 0 355 047
- EP-A- 1 748 063
- US-A1- 2003 166 869
- GODFREY M A J ET AL: "A SENSITIVE ENZYME-LINKED IMMUNOSORBENT ASSAY (ELISA) FOR THE DETECTION OF STAPHYLOCOCCAL PROTEIN A (SPA) PRESENT AS A TRACE CONTAMINANT OF MURINE IMMUNOGLOBULINS PURIFIED ON IMMOBILIZED PROTEIN A" JOURNAL OF IMMUNOLOGICAL METHODS, ELSEVIER SCIENCE PUBLISHERS B.V.,AMSTERDAM, NL, vol. 149, no. 1, 27 April 1992 (1992-04-27), pages 21-27, XP008042183 ISSN: 0022-1759
- HORENSTEIN A L ET AL: "Design and scaleup of downstream processing of monoclonal antibodies for cancer therapy: from research to clinical proof of principle" JOURNAL OF IMMUNOLOGICAL METHODS, ELSEVIER SCIENCE PUBLISHERS B.V.,AMSTERDAM, NL, vol. 275, no. 1-2, 1 April 2003 (2003-04-01), pages 99-112, XP004416758 ISSN: 0022-1759
- BEER D J ET AL: "A comparison of the leakage of a monoclonal antibody from various immunoaffinity chromatography matrices" BIOSEPARATION, vol. 5, no. 4, 1995, pages 241-247, XP009072651 ISSN: 0923-179X
- MERTEN O-W: "DEVELOPMENT OF SERUM-FREE MEDIA FOR CELL GROWTH AND PRODUCTION OF VIRUSES/VIRAL VACCINES - SAFETY ISSUES OF ANIMAL PRODUCTS USED IN SERUM-FREE MEDIA" DEVELOPMENTS IN BIOLOGICALS, KARGER, BASEL, CH, vol. 111, 2002, pages 233-257, XP009045722 ISSN: 1424-6074
- GOLDSTEIN I ET AL: "ACTION OF GROUP A STREPTOCOCCUS EXTRACELLULAR PRODUCT ON THE CONNECTIVE TISSUE OF THE BOVINE HEART VALVE" INFECTION AND IMMUNITY, vol. 9, no. 1, 1974, pages 20-26, XP002400310 ISSN: 0019-9567
- PENG Z K ET AL: "Differential binding properties of protein A and protein G for dog immunoglobulins." JOURNAL OF IMMUNOLOGICAL METHODS 15 DEC 1991, vol. 145, no. 1-2, 15 December 1991 (1991-12-15), pages 255-258, XP002415156 ISSN: 0022-1759

## Description

### FIELD OF THE INVENTION

This invention relates to methods for the purification of vegetarian (non-animal derived) Protein A using a multidimensional purification process to remove undesirable non-animal derived component impurities in the Protein A from the non-animal fermentation media used to produce the Protein A so as to produce a vegetarian Protein A free of animal-origin components and essentially free of components derived from non-animal derived growth media.

### BACKGROUND OF THE INVENTION

Protein A is a cell wall protein made by *Staphylococcus aureus*. The immunoglobulin-binding protein of the Gram-positive *Staphylococcus aureus*, Protein A, binds strongly to the Fc region of immunoglobulins from human, guinea pig, pig, and rabbit. See Langone, (1982) Adv. Immunol. 32:157 and Lindmark (1983) J. Immunol. Methods 62:1. Protein A binds, to a lesser extent, to immunoglobulin M (IgM) having a V_{H3} region, without affecting immunoglobulin affinity for antigen. Protein A is used commercially to purify monoclonal antibodies that are often in turn used as therapeutics in human diseases like inflammatory diseases and cancer. Also, Protein A can itself be used as a therapeutic. Protein A can be administered to a patient in order to bind circulating immune complexes in autoimmune diseases like rheumatoid arthritis or to stimulate specific cytokine production in someone with an infection. In addition, when coupled to a chromatography resin, Protein A can act as a therapeutic absorber to treat plasma or whole blood by removing IgG complexes in disorders like autoimmune disease and transplant organ rejection.

In some strains of *S*. *aureus* the Protein A remains bound to the cell wall while in some others (secretors), Protein A is released as a soluble protein product of molecular weight 40-55,000 into the growth media. See Lindmark, (1977) Eur. J. Biochem. 74:623. Protein A is extensively used in the commercial production of monoclonal antibodies. Soluble and functional Protein A and functional fragments of Protein A representing several of the 5 IgG binding domains of Protein A have been produced by molecular cloning, all of which demonstrated affinity for IgG. Typically, the clones are expressed in bacteria which have been grown in media partially or wholly derived from animal sources, such as bovine milk caseins, mammalian meat stocks or broths.

Originally, Protein A was derived from the *S. aureus* cell wall, but bacterial strains which secrete the protein have been isolated and are also used to obtain Protein A. However, the preparation of Protein A by conventional methods presents a problem for its use in human therapeutics. *S aureus* is grown in media that contains animal products as a source of amino acids for the bacteria. In particular, bovine hydrolysates and extracts are commonly used as supplements in bacterial growth media. Hence, obtaining Protein A from growth media containing animal-derived products leads to the possibility that animal products like prions, the causative agent in Mad Cow, Scraple and wasting disease, could be present in the media and transmitted to human patients. Protein A preparations can also be exposed to and contaminated with animal products during downstream purification processes. For example, in methods currently used, Protein A is purified using an IgG Sepharose chromatography column and the IgG coupled to the Sepharose chromatography column is typically of animal origin. However, the animal products that come to be associated with a Protein A preparation during the isolation and purification processes are not easily purified from the preparation by the methods presently known.

Recent health concerns related to the transmission of various wasting diseases, such as bovine spongiform encephalopathy (BSE), one of several diseases categorized as transmissible spongiform encephalopathy (TSE) and variant Creutzfeldt-Jacob disease (CJD), prion-related diseases, has prompted the removal of animal-derived materials from bacterial growth media where any product obtained from bacteria growing in such media might come in contact with humans. Thus, vegetable-derived media has begun replacing animal-derived media for growing microbes which are designed to produce a product that ultimately becomes a medicinal agent, such as vitamins, amino acids, or therapeutic proteins, such as antibodies. Hence, there is the need for a commercially viable means to manufacture and purify a non-animal derived Protein A for use in therapeutic antibodies that must have high purity, binding properties at least equal to commonly used Protein A and assurance of animal-free growth conditions and purification processes.

Purification processes developed long ago for isolating products from animal-derived microbial fermentations were designed to remove unwanted components produced by the microbe in the fermentation process or fortuitously added as a part of the growth media and either altered by the microbe or left unaltered. Years of practice in the art of purification led to numerous processes to accomplish such purifications. However, in cases such as the isolation of a soluble protein secreted by the microbe into the medium, much of the purification process revolved around removing the media components which were added in high levels to promote rapid growth and efficient production of the product of interest. In the event of a switch of media ingredients, a new spectrum of contaminants will likely appear, thus, there will be the need to remove them from the product of interest. However, they may not purify out by the protocols developed for isolations from previous media. Hence, the change from animal-derived growth media to non-animal-derived media components introduces a variety of challenges to the purification process, such as differences in solubilities of media components, carbohydrate content, lipid content, protein content, particulates, viscosity, etc. Each of these have bearing on the speed, efficiency and effectiveness of the process developed originally for animal-derived growth media fermentations and growth.

Protein A derived from aimal origin is normally purified by either of two methods, a "classical" method using some combination of ion exchange columns and/or size exclusion columns or affinity columns, employing a purified immunoglobulin (animal derived) attached covalently to an insoluble matrix such as Agarose or silica. In order to produce a Protein A product that is free of animal-derived contaminants, it is necessary to remove any animal-derived product from its recent history and from the fermentation process and also from the purification process. Thus, it becomes necessary to isolate a stock culture of the growth organism in uniquely non-animal-derived culture media, grow the microbe in non-animal-derived media and purify the Protein A product using non-animal-derived media to such an extent that it is free of contaminating bacterial proteins and components derived from the media.

Attempts to purify Protein A by classical methods from *S*. *aureus* grown on conventional animal-derived media gave a typical protein product with high purity as determined by SDS gel electrophoresis and analytical chromatography with a typical UV spectrum for Protein A (See Sjoquist, (1972) Eur. J. Biochem. 29:572). At concentrations of 1-10 mg/mL, the solution was nearly colorless. However, in our initials trials using *S*. *aureus* grown on non-animal-derived media and purified by the same process, the Protein A product had a yellow color and gave a substantial absorbance in the high UV and visible region (300-450 nm), but gave the same pattern on SDS gel electrophoresis, indicating high purity. This suggested there were non-protein contaminants present. Indeed, 1-5 mg/mL solutions of such a Protein A were a distinct yellow brown, a color resembling a diluted version of the growth media. Thus it appeared that the extant purification procedures were not removing all the non-animal derived growth media components. Thus there developed a need to provide a new, improved purification process for purifying vegetarian derived Protein A.

### SUMMARY OF THE INVENTION

This invention provides a non-animal derived Protein A purification process that comprises:
(a) providing a non-animal derived Protein A solution that has been produced by fermenting a secretor strain of *Staphylococcus aureus* in a media containing non-animal derived amino acids or peptides, said media being free of animal products;
(b) filtering said Protein A solution to remove bacterial cells, particulates and low molecular weight molecules (< 10,000 daltons) before loading the Protein A solution onto a first column;
(c) loading the filtered Protein A solution onto the first column;
(d) washing the column with a buffer-salt mixture to rinse off the column undesirable color and contaminating proteins;
(e) eluting the Protein A from the first column to produce a Protein A solution pool;
(f) adjustment of the pH and conductivity of the Protein A solution pool;
(g) loading the Protein A from the Protein A solution pool onto a second column;
(h) washing the second column with a buffer-salt mixture to rinse off the column undesirable color and contaminating proteins; and
(i) eluting the Protein A from the second column to produce a purified Protein A solution;
wherein the first column is a cation exchange column and the second column is a column selected from the group consisting of an anion exchange column, a HIC column and a ceramic hydroxyapatite column, and
wherein the Protein A has a purity of >95% by SDS gel electrophoresis and a pool absorbance ratio at 275nm:340nm of greater than 7.0.

There is disclosed herein a bacterial fermentation in non-animal-derived growth media to give a high concentration of Protein A in the exudates which is purified using diafiltration and columns to yield a product that is >95% pure by SDS gel electrophoresis and by chemical and physical tests is substantially Identical with Protein A purified from spent culture media of animal-derived origin. Further, contaminants left over from the non-animal growth medium have virtually been eliminated. In one instance a seed culture of *S*. *aureus var. IMRE* known to secrete Protein A was serially grown through 2 culturings to eliminate animal-derived media carry-over in the seed culture. Seed culture stocks were transferred to approximately 400 sterile culture vials and maintained at -70° C until needed. Seed cultures were used to inoculate 2 liters of non-animal derived media composed of 1.8% Soy Peptone (Sigma), 5% yeast extract (Sigma), 0.5% sodium chloride (Mallinckrodt Baker), 0.25% dextrose, 0.25% potassium phosphate, dibasic (Fisher), in water and NaOH to render pH 7.5. Growth at 37° C was encouraged with aeration and pH maintenance by additions of 1 M phosphoric acid or sodium hydroxide for 16 hours, then this innoculum was transferred to a 16 liter growth chamber containing the same composition of media and growth was continued at 37° C with aeration at 16 l/minute for at least 10 hours or until the absorbance units at 560 nm reached 52. Then, a larger run may be made by using this 16 liter culture as innoculum for a larger, say 375 liter, fermentor containing the same medium previously sterilized and again allow growth at 1 Liter air per minute per liter of media for sufficient time to reach approximately 50 absorbance units at 560 nm. Cells were removed by centrifugation or by filtration through 0.1 micron filters and the broth/culture media was then filtered through a Millipore PROCON 10,000 MWCO TFF membrane to retain the Protein A, reduce the salt concentration and concentration of lower molecular weight components and to exchange the buffer. The resultant concentrated protein/broth solution was then applied to an appropriate column which was then washed with a buffered solution then the Protein A was eluted in either a gradient or step elution with higher salt concentrations. A pool was made of active fractions of appropriate purity and either simply diluted with water to an appropriate salt concentration or diaflow treated to concentrate and exchange buffers. This protein solution was then applied to a second column which was washed, then the Protein A eluted with a buffered higher salt concentration and fractions pooled to obtain Protein A at a recovery of 80-100%, purity > 95% by HIC, purity >95% by SDS gel electrophoresis and such that the pool absorbance ratio at 275nm:340nm was greater than 7.0. Appropriate columns might be either an anion exchange matrix, a ceramic hydroxyapatite matrix, hydrophobic matrix or a cation exchange matrix. The anion exchange matrix may be any of the useful anion exchange resins though the preferred embodiment of the invention is XWP 500 PolyQuat-35 (Mallinckrodt Baker # 7603) pre-equilibrated with 20 mM Tris-Cl, pH 7.8. The preferred ceramic hydroxyapatite matrix (Bio-Rad) was pre-equilibrated with 20 mM Tris-Cl, pH 7.8. The cation exchange matrix may be any of the useful cation exchange resins though the preferred embodiment of the invention Is WP carboxy-sulfone (weak-strong cation exchanger) (Mallinckrodt Baker # 7587) pre-equilibrated with 137 mM acetic acid-triethanolamine-30 mM NaCl, pH 4.5-6.0. Typically, after the second ion exchange column, the Protein A solution may be dialyzed or diafiltered to remove the bulk of the salt and buffer components then frozen and lyophilized.

### DETAILED DESCRIPTION OF THE INVENTION

Protein A can be obtained from any Protein A-producing strain of *S*. *aureus* Including those strains that are naturally-occurring, isolated or genetically engineered. Protein A-producing strains of S. *aureus* can also include those strains in which Protein A remains embedded In the cell wall and those strains that secrete Protein A into the growth media, preferably *S. aureus*, var Imre, for Instance. Methods by which Protein A can be recovered from the *S. aur*e*us* cell wall (e.g., enzyme digestion) are well known by those with skill in the art. Protein A recovered in this manner can be purified to eliminate the digested cellular components. It is preferable, however, to obtain Protein A from *Staphylococcus* strains that secrete Protein A into the media such that it is only necessary to harvest the Protein A from the media.

Seed cultures designed to be free of animal-source nutrients and components were obtained from stocks of *S. aureus* by serial plating on media of microbiological-derived and vegetable-derived components and nutrients. Several media compositions gave good growth with varying amounts of Protein A in the exudates, especially dependent on the yeast extract content of the medium. A disadvantage of the non-animal derived media is the greater color intensity produced in the initial Protein A solution. Initial efforts to purify the Protein A using previously adopted means gave a product with a substantial amount of color compared to Protein A purified in the same manner from less colorful animal-derived media. Employing the multidimensional exchange column process of this invention significantly reduced or substantially eliminated this impurity coloration. Protein A eluted from a second chromatography column in accordance with the process of this invention substantially reduced color content giving a Protein A of high purity (>98% by SDS gels) that was essentially identical physically and chemically to Protein A isolated from animal-derived media sources.

As referred to herein, "animal products" are defined as any material transmitted or derived from a non-human animal, the material including peptides, proteins, amino acids, protein-like materials, nucleic acids, viruses and the like. As referred to herein, the term "vegetarian Protein A" is defined as a Protein A composition uncontaminated by animal products or any animal-derived peptides that are derived from the fermentation media or the purification media. Similarly, the term "vegetarian media" refers to fermentation media uncontaminated by animal products or any animal-derived peptides. As used herein, the term "vegetarian amino acids or peptides" refers to any peptides, proteins, amino acids or protein-like materials that are not derived from a non-human animal.

The following is a brief description of the materials and methods employed. The materials and methods are illustrative of, but not limited to, those materials and methods that may be employed in the process described herein.

Stock of Seed cultures derived from *S*. *aureus* var. IMRE. Secretor bacteria can be fermented in any amount of vegetarian media sufficient to grow the bacteria and produce the desired amount of Protein A. Most preferably, the *S*. *aureus* are grown in a large scale manufacturing batch in fermentor tanks. Fermentation conditions (i.e., airflow, temperature, agitation, pH and pressure) can be controlled and/or monitored electronically, with, for example, a microprocessor, or by any other electronic means. Some particularly preferred conditions include fermentation of the *S*. *aureus* at about 37° and an airflow of about a liter/minute of sterile air per liter of media. The media can consist of the components of conditioned media for *S*. *aureus* growth that are known in the art, typically, yeast extract, glucose and essential salts, and, in the case of the invention, includes vegetarian amino acids and peptides (e.g., soy, pea hydrolyzate or cotton seed hydrolyzate peptide) as a source of amino acids. Vegetarian amino acids or peptides could, for instance, be derived from any non-animal (e.g. plant or plant-like) or synthetic (e.g., chemically synthesized) source and in one embodiment of the invention, the vegetarian amino acids or peptides are derived from soy. The components can be at a concentration of about 50 grams/liter (g/L) yeast extract, 15 g/L soy peptides and 5 to 10 g/L glucose dissolved in purified, deionized water with diluted phosphoric acid and diluted sodium hydroxide added as needed during fermentation to control the pH at between about 7 and 8. The media can further contain at least 0.67 milliliters/liter (mL/L) of polyethylene glycol to prevent foaming in the fermentation mixture. Preferably, a small culture of *S. aureus var*. *IMRE* was streaked onto sterile agar media composed of non-animal-derived components: 1.8% Soy Peptone (Sigma), 5% yeast extract (Sigma), 0.5% sodium chloride (Mallinckrodt Baker), 0.25% dextrose, 0.25% potassium phosphate, dibasic (Fisher), 3% agar (Difco) in water and NaOH to render pH 7.5. The plate was incubated at 37° C overnight. Using a single colony from this plate, a transfer was made to another plate of the same composition by streaking and allowed to grow under the same conditions overnight. A single colony from this second streak was transferred to 100 mL of sterile non-animal-derived liquid media sterilized by autoclaving at 121 °C for 25 minutes: 1.8% Soy Peptone (Sigma), 5% yeast extract (Sigma), 0.5% sodium chloride (Mallinckrodt Baker), 0.25% dextrose and 0.25% potassium phosphate, dibasic (Fisher) in water and NaOH to render pH 7.5. This liquid culture was agitated at 300 RPM overnight at 37 °C. Ten mL of this liquid culture was then used to inoculate a 16 liter fermentor containing 12 liters of non-animal derived media sterilized by autoclaving: 3% Select APS^{™} TSB (Becton-Dickinson and Co.) and 0.08% polyethylene glycol. The culture was grown at 37°C with aeration at about 12 L/minute while stirring at 400 RPM for at least 10 hours or to a cell density of 52 absorbance units at 560 nm. The pH was maintained at 7.0 +/- 0.5 with dilute NaOH and dilute phosphoric acid. Portions of the cultured broth were centrifuged at 2000 x g for 30 minutes to concentrate the cells. The concentrated cell mass was suspended with approximately 4 volumes of media sterilized by autoclaving: 3% Select APS^{™} TSB (Becton-Dickinson and Co.) and 0.08% polyethylene glycol. Approximately 4-5 mL portions of the cell suspension were transferred into each of about 400-500 sterile vials and stored at -70°C until needed. Approximately 5% of the vials were tested to verify bacterial purity. Additionally, The fermentation broth can be assessed visually under a microscope to ensure the broth is not contaminated with other microorganisms. Similarly, the *S*. *aureus* in the culture can be inspected using a microscope for the proper morphology which includes having: the shape of gram positive cocci, pleiomorphism (i.e., single colonies, chains or roseates) and a size of approximately 0.6 microns. The cultured *S. aureus* can also be assessed for quality and health by determining if they exhibit a round, raised, grayish morphology on blood agar plates, have an ability to cause the β-hemolysis of sheep blood after 48 hours and test positive for the enzymes coagulase and catalase.

Desalting of the Column-eluted Protein A solution. Once the pool is obtained from the second column in the series, dialyze or diafilter against deionized or distilled water until essentially salt-free (conductivity less than 2 mS/cm). Dialysis membranes may include tubing membranes or cassettes containing walls made of cellulose membranes with MWCO <10,000 treated according to the manufacturer's recommendations. Dialyzed or diafiltered protein solutions may be frozen in bottles in a rotating device that promotes the freezing of the solution onto the walls of the bottle in a shell or may be placed in shallow glass pans on a plate or shelf at below freezing temperatures. In this embodiment, freezing is done in large glass Petri plates on a shelf set at -55° C until the temperature of the frozen solution has reached -15° C or lower at which time evacuation is begun with a vacuum pre-set to 200-250 mTorr and the shelf is set to -15° C. Freeze-drying is continued for 2-24 hours at which time the shelf temperature is raised to 0°C until the product is visibly dry at which time the shelf temperature is raised to 23°C and held for at least 2 hours for final drying at which time the dry white to light tan fluffy product is removed and may be kept refrigerated or in a freezer.

Analytical C₄ (HIC) Column. A WP Butyl (C₄) Reverse Phase 4.6 X 250 mm column (Mallinckrodt Baker # 7116-00) was thoroughly pre-equilibrated with water:acetonitrile:trifluoroacetic acid (80:20:0.1) (volumes) at 1.0 mL/minute using a Waters 515 HPLC Pump. Samples or standards were dissolved or diluted with water to 0.1 - 1.5 mg/mL and 0.025 mL injected and the effluent monitored at 214, 275 and 340 nm (Waters 996 Photodiode Array Detector). Immediately after injection, a linear gradient was begun starting with equilibration buffer and ending at 30 minutes with a 50:50 mix of equilibration buffer and elution buffer (elution buffer is composed of water:acetonitrile:trifluoroacetic acid at 25:75:0.1). The column was rinsed for 1 minute with elution buffer (100%) then re-equilibrated with equilibration buffer for 35 minutes before the next sample was injected. Areas under absorbance peaks at 214 nm were determined by the instrument. Standard samples of Protein A were made by dissolving 5 to 25 mg of dry purified Protein A in water to a concentration of 1.5 mg Protein A/mL and storing 0.35-0.85 mL in microcentrifuge tubes at -10 to -15° C until needed. A stock tube of 1.5 mg Protein A/mL frozen solution was thawed and diluted 1:1, 1:3, 1:7 and 1:15 with water to generate a set of concentration standards. Protein A typically eluted at 15-17 minutes. Areas under the standard Protein A peaks at 214 nm were plotted against the Protein A concentration of the standard applied to generate a standard curve. Areas obtained from test samples were compared with the standard curve to obtain the concentration of the sample. If the area of the sample exceeded the area obtained for the 1.5 mg/mL standard, the sample was diluted with water appropriately and run again.

Analytical Size Exclusion Column. A standard size exclusion column (7.8 x 300 mm TSK-Gel G3000 SW (Tosoh)) was equilibrated with 0.1 M sodium phosphate, pH 7.0, containing 0.5 M NaCl. Typically, samples of 0.10 mL were applied and run at 0.5 mL/minute (Waters 515 HPLC Pump) while monitoring the effluent at 214, 280 and 340 nm (Waters 996 Photodiode Array Detector). Areas under absorbance peaks at 214 nm were determined by the instrument. For quantitation of concentration, standard samples of Protein A were made by dissolving 5 to 25 mg of dry purified Protein A in water to a concentration of 1.5 mg Protein A/mL and storing 0.35-0.85 mL aliquots in microcentrifuge tubes at -10 to -15° C until needed. A stock tube of 1.5 mg Protein A/mL frozen solution was thawed and diluted 1:1, 1:3, and 1:7 with water to generate a set of concentration standards. Protein A eluted at 15-16 minutes. Areas under the standard Protein A peaks at 214 nm were plotted against the Protein A concentration of the standard applied to generate a standard curve. Areas obtained from test samples were compared with the standard curve to obtain the concentration of the sample. If the area of the sample exceeded the area obtained for the 1.5 mg/mL standard, the sample was diluted with water appropriately and run again. For molecular weight estimation, standard test proteins were dissolved in 20 mM Tris-Cl, pH 8 to give 1.0 mg/mL and 0.1 mL injected and run as above for the concentration standards. For molecular weight estimation, a standard curve was obtained by plotting the log₁₀ of the known molecular weight standard against the retention time of the standard. Retention times of protein peaks monitored at 214 nm or 280 nm in the samples were compared with the standard curve to obtain the molecular weight estimate of the sample's proteins. Protein A elutes at a position giving an estimated molecular weight of 85,000, considerably higher than the expected value of 40-50,000 from the known structure and data obtained by SDS gel electrophoresis. This is likely related to the non-spherical nature of the native protein (Bjork, (1972) Eur. J. Biochem. 29:579).

Denaturing Gel Electrophoresis. Solutions containing protein were diluted or dissolved with 1% sodium dodecyl sulfate-0.1 M dithiothreitol-6 M urea-0.125 M Tris-HCl, pH 6.8, to a final protein concentration of 0.1-1.0 mg/mL then heated to 65°C for 5-10 minutes and cooled to room temperature. Protein standards are obtained in a kit form from Sigma Chemical Co. or Bio-Rad. Between 1 and 25 microliters of protein solution may be applied to a polyacrylamide gel made commercially for the purpose of protein subunit (monomer) size determination such as 4-20% Ready Gels (Bio-Rad), 4-20% iGels (Gradigels), 4-20% Novex Pre-cast Gels (Invitrogen) or gels may be made as 12% or greater in acrylamide/bis-acrylamide (37.5/1 ratio) according to Laemmli (Laemmli, (1970) Nature 227:680). Following the instructions of the manufacturer or the Laemmli procedure and at the end of the electrophoresis, the gel is fixed, stained and destained by any of several commonly used methods employing 40% methanol-7% acetic acid-water and either 0.1% coomassie blue R250 or silver staining technique, depending on the sensitivity needed. Kits are commercially available for silver staining (Sigma or Bio-Rad).

Ultraviolet Spectral Properties of Protein A Isolated from Animal-derived and Non-animal-derived Media. Background absorbancies were taken on water in a 1 cm pathlength quartz cuvette on a Perkin-Elmer λ800 from 200 to 400 nm. Similarly, absorbancies were taken at 200-400 nm on protein samples with the background subtracted. If the absorbance at any wavelength in the 400 to 250 nm region exceeded 2.0 absorbance units, the sample was diluted with 2 equal parts of water and tested again.

Absorbance Ratios at 275 and 340 nm. A spectrophotometer was blanked with water or buffer in which the Protein A may be dissolved in a quartz cuvette of 1 cm path length to zero the instrument at 275 nm. Absorbance of any protein solution in question is read and recorded at the same wavelength in the same quartz cuvette. If it is recognized that the buffer in which the protein is dissolved absorbs light at either 275 nm or 340 nm, then the buffer in question should be used to blank the instrument rather than water. This process is repeated at 340 nm. The ratio of the absorbance at 275 nm to the absorbance at 340 nm is recorded as A275/A340 and for any Protein A eluted from a second column should exceed 7.0.

Lyophilization of Purified Protein A. Protein A eluted from the second purification column is diafiltered against water or dialyzed against water to remove the bulk of salts and low molecular weight components. This solution is then frozen onto the sides of rotating bottles so as to form a shell of frozen liquid or frozen rapidly into large cold glass pans. The frozen solutions are cooled on a shelf set at -55° C until the temperature of the frozen solution has reached -15° C or lower at which time evacuation is begun with a vacuum pre-set to 200-250 mTorr and the shelf is set to -15° C. Freeze-drying is continued for 2 to 24 hours at which time the shelf temperature is raised to 0°C until the product is visibly dry at which time the shelf temperature is raised to 23°C and held for at least 2 hours for final drying, at which time the dry white to light tan fluffy product is removed and may be kept refrigerated or in a freezer.

### EXAMPLES

(1) Large-scale Growth of *S*. *aureus.* One vial of the seed culture was grown in non-animal-derived growth media in a 16 liter fermentor containing sterile media composed of 50 g/L yeast extract (Difco), 15-20 g/L soy peptone (Sigma), 5 g/L NaCl (Integra), 2.5-25 g/L Glucose (Fluka), 2.5 g/L potassium phosphate, dibasic (Mallinckrodt Baker), 1mL Anti-Foam (Dow)/liter of media and water with pH adjusted to 7.5-7.8. Growth at 37° C was encouraged with aeration at 1 Liter/liter of media and pH maintenance at 7.5-7.8 was affected by additions of 1 M phosphoric acid or dilute sodium hydroxide for approximately 16-22 hours, then this innoculum was transferred to a 400 liter growth chamber containing the same composition of sterile media and growth was continued at 37° C with aeration at 1 L/minute/Liter of medium and stirring at 800 RPM for at least 10 hours to a cell density of at least 50 absorbance units at 560 nm. At this point, the production of Protein A free in the media in a secreted form is approximately 0.2-1.0 mg Protein A/mL. Cells were removed by centrifugation or by filtration through 0.1 or 0.2 micron filters and the broth/culture media filtrate was then diafiltered through a Millipore PROCON 10,000 MWCO TFF membrane to retain the Protein A, reduce the salt concentration and concentration of lower molecular weight components, to reduce the volume of the solution and to exchange the buffer to one appropriate to the column to which it is to be applied. Appropriate columns might be either an anion exchange matrix, a ceramic hydroxyapatite matrix, a hydrophobic matrix or a cation exchange matrix. The anion exchange matrix may be any of several anion exchange resins such as WP DEAM (weak) or WP PEI (Mallinckrodt Baker) though the preferred embodiment of the invention is XWP 500 PolyQuat-35 pre-equilibrated with 20 mM Tris-Cl, pH 7.8 using a Waters 515 HPLC Pump. The ceramic hydroxyapatite matrix (Bio-Rad) was pre-equilibrated with 20 mM Tris-Cl, pH 7.8. The hydrophobic matrix may be any of several short-chain aliphatic materials attached covalently to silica, such as ethyl, propyl, isopropyl, butyl, iso-butyl or pentyl-silica equilibrated with 20 mM Tris-HCl, pH 7.8, and containing 0.5 M ammonium sulfate. The cation exchange matrix may be any of several cation exchange resins though the preferred embodiment of the invention is WP carboxy-sulfone (weak-strong cation exchanger) (Mallinckrodt Baker # 7587) and is equilibrated with 137 mM acetic acid-triethanolamine-30 mM NaCl, pH 4.5-6.0.

### (2) Application of Protein Solution to Anion Exchange Column First

For application of the Protein A solution to the anion exchange column, the solution from 375 liters of fermentation broth was diafiltered through a Millipore PROCON 10,000 MWCO TFF membrane to retain the Protein A, reduce the salt concentration, reduce the concentration of lower molecular weight components, to exchange the buffer to 20 mM Tris-Cl, pH 7.8 and to reduce the volume of the solution. This protein solution was applied to a 10 X 75 cm XWP 500 PolyQuat-35 (Mallinckrodt Baker # 7603) with a Waters 515 HPLC Pump at a flow rate of 5.5 - 8.0 cm/minute. Non-bound components were washed off the column with 3-10 column volumes of fresh equilibration buffer, then the Protein A was eluted from the column with 20 mM Tris-Cl, pH 7.8 adjusted to a conductivity of 18 mS/cm with NaCl. Protein A concentration and purity were determined on fractions using an analytical C₄ (HIC) reverse phase column and by monitoring absorbance at 214, 275 and 340 nm on a Waters Photodiode Array Detector. Fractions of Protein A are pooled such that the recovery is 85-100% and the pool A275/A340 ratio is 7 or higher. At this point, the protein solution is then applied to a cation exchange column. For the cation exchange, the Protein A pool was titrated to pH 4.5-6.0 with dilute HCl and then diluted with water to a conductivity of 4.0-7.3 mS/cm and applied to a 16 x 72 cm WP carboxy-sulfone column (weak-strong cation exchanger) pre-equilibrated with 137 mM acetic acid-triethanolamine-30 mM NaCl, pH 4.5-6.0 with pH 4.7, being the preferred embodiment, at a flow rate of 100-300 cm/hour. The column was then washed at the same flow rate with 2-10 column volumes of equilibration buffer followed by elution of the Protein A with a step of 137 mM acetic acid-triethanolamine-200 mM NaCl, pH 4.5-6.0 or gradient of 0-1.0 M NaCl in the equilibration buffer over a 10 column volume range. Protein A concentration and purity were determined on fractions using an analytical C₄ (HIC) reverse phase column and by monitoring absorbance at 214, 275 and 340 nm on a Waters Photodiode Array Detector. The A275 and A340 were examined on Protein A-containing fractions and then they were pooled as those fractions having A275/A340 ratios exceeding 7.0.

(3) Application of Protein Solution to Cation Exchange Column First. For application of the Protein A solution to the cation exchange column, the solution from 375 liters of fermentation broth was diafiltered through a Millipore PROCON 10,000 MWCO TFF membrane to retain the Protein A, reduce the salt concentration and concentration of lower molecular weight components, reduce the volume of solution and to exchange the buffer to 137 mM acetic acid-triethanolamine-30 mM NaCl, pH 4.5-6.0, the preferred pH being 4.7. Alternatively, the protein solution may simply be filtered through a 0.2 micron filter, pH adjusted to 4.7-6.0 with 1 N HCl or 1 N NaOH, and diluted with water to a conductivity of 4.0 - 7.0 mS/cm. This protein solution was applied to a 16 x 72 cm WP carboxy-sulfone column at a flow rate of 100-300 cm/minute. Non-bound components were washed off the column with 3-10 column volumes of equilibration buffer followed by elution of the Protein A with a step of 137 mM acetic acid-triethanolamine-200 mM NaCl, pH 4.5-6.0 or gradient of 0-1.0 M NaCl in the equilibration buffer over a 10 column volume range. Protein A concentration and purity were determined on fractions using an analytical C₄ (HIC) reverse phase column and by monitoring absorbance at 214, 275 and 340 nm on a Waters Photodiode Array Detector. The A275 and A340 were examined on Protein A-containing fractions and those were pooled having A275/A340 ratios exceeding 7.0. At this point, a choice can be made to apply the protein solution to an anion exchange, hydrophobic interaction or ceramic hydroxyapatite column, the preferred embodiment was an anion exchange column, whereupon the Protein A pool was diafiltered through a Millipore PROCON 10,000 MWCO TFF membrane to retain the Protein A, reduce the salt concentration, reduce the concentration of any remaining lower molecular weight components, to exchange the buffer to 20 mM Tris-Cl, pH 7.8 and to reduce the volume of the solution. This protein solution was then applied to a 10.5 X 72 cm column of XWP 500 PolyQuat-35 anion exchange resin (Mallinckrodt Baker #7603) pre-equilibrated with 20 mM Tris-Cl, pH 7.8 using a Waters 515 HPLC Pump at a flow rate of 100-250 cm/hour. Non-bound components were washed off the column with 3-10 column volumes of fresh equilibration buffer, then the Protein A was eluted from the column with 20 mM Tris-Cl, pH 7.8 adjusted to a conductivity of 18 mS/cm with NaCl. Protein A concentration and purity were determined on fractions using an analytical C₄ (HIC) reverse phase column and by monitoring absorbance at 214, 275 and 340 nm on a Waters Photodiode Array Detector. Fractions of Protein A were pooled such that the recovery is 85-100% and the A275/A340 ratio is 7.0 or higher. Alternatively, if the HIC column is the second column choice, the pool is mixed with sufficient granular or powdered ammonium sulfate to render a final concentration of 0.5 M ammonium sulfate and the pH adjusted to 7.5 - 8.2 with dilute ammonium hydroxide. This solution is applied to a 16 x 72 cm column of WP HI-Propyl (C₃) polymeric resin pre-equilibrated with 0.5 M ammonium sulfate in 20 mM sodium phosphate, pH 8.0. The column is washed with 2-5 column volumes of 0.5 M ammonium sulfate in 20 mM sodium phosphate, pH 8.0, then begin a gradient of 5 volumes of 0.5 M ammonium sulfate in 20 mM sodium phosphate, pH 8.0 to 5 volumes of 20 mM sodium phosphate, pH 8.0. Protein A concentration and purity are determined on fractions using an analytical C₄ (HIC) reverse phase column and by monitoring absorbance at 214, 275 and 340 nm on a Waters Photodiode Array Detector. A275 and A340 are examined on fractions containing Protein A and pooled as those with 275/340 ratios exceeding 7.0. If the choice for the second column is a ceramic hydroxyapatite, the protein solution is diafiltered to exchange the buffer to 20 mM Tris-HCl, pH 7.5. A 16 x 72 cm column of ceramic hydroxyapatite (Bio-Rad) is equilibrated witch 20 mM Tris-HCl, pH 7.5 and the protein solution is loaded onto the column at 100-150 cm/hour. The flow is continued with a wash of the same buffer for 3-5 column volumes then Protein A eluted with 3 column volumes of 20 mM Tris-HCl, pH 7.5, containing 0.2 M ammonium sulfate. Protein A concentration and purity are determined on fractions using an analytical C4 (HIC) reverse phase column and by monitoring absorbance at 214, 275 and 340 nm on a Waters Photodiode Array Detector. Examine A275 and A340 on fractions and pool those Protein A-containing fractions with A275/A340 ratios of greater than 7.0.

(4) Application of Protein Solution to Anion Exchange Column Followed by a Cation Exchange Column. In a preferred embodiment, the Protein A solution is applied first to the anion exchange column. The solution from 375 liters of fermentation broth was diafiltered through a Millipore PROCON 10,000 MWCO TFF membrane to retain the Protein A, reduce the salt concentration, reduce the concentration of lower molecular weight components, to exchange the buffer to 20 mM Tris-Cl, pH 7.8 and to reduce the volume of the solution. This protein solution was applied to a 10.5 X 72 cm column of XWP 500 PolyQuat-35 with a Waters 515 HPLC pump at a flow rate of 5.5 - 8.0 cm/minute. Non-bound components were washed off the column with 3-10 column volumes of fresh equilibration buffer, then the Protein A was eluted from the column with 20 mM Tris-Cl, pH 7.8 adjusted to a conductivity of 18 mS/cm with NaCl. Protein A concentration and purity were determined on fractions using an analytical C₄ (HIC) reverse phase column and by monitoring absorbance at 214, 275 and 340 nm on a Waters Photodiode Array Detector. Fractions of Protein A were pooled such that the recovery is 85-100% and such that the pool exhibited an A275/A340 ratio of 7.0 or higher. The Protein A pool was titrated to pH 4.5-6.0 with dilute HCl and then diluted with water to a conductivity of 4.0-7.3 mS/cm and applied to a 16 x 72 cm WP carboxy-sulfone column (weak-strong cation exchanger) pre-equilibrated with 137 mM acetic acid-triethanolamine-30 mM NaCl, pH 4.5-6.0 with pH 4.7 being the preferred embodiment, at a flow rate of 100-300 cm/hour. The column was then washed at the same flow rate with 2-10 column volumes of equilibration buffer followed by elution of the Protein A with a step of 137 mM acetic acid-triethanolamine-200 mM NaCl, pH 4.5-6.0 or gradient of 0-1.0 M NaCl in the equilibration buffer over a 10 column volume range. Protein A concentration and purity were determined on fractions using an analytical C₄ (HIC) reverse phase column and by monitoring absorbance at 214, 275 and 340 nm on a Waters Photodiode Array Detector. A275 and A340 of fractions was examined on all Protein A-containing fractions and those with A275/A340 ratios exceeding 7.0 were pooled.

(5) Application of Protein Solution to Cation Exchange Column Followed by an Anion Exchange Column. In another preferred embodiment, the Protein A solution was applied to a cation exchange column WP carboxy-sulfone (weak-strong cation exchanger) (Mallinckrodt Baker # 7587). The solution from 375 liters of fermentation broth was diafiltered through a Millipore PROCON 10,000 MWCO TFF membrane to retain the Protein A, reduce the salt concentration and concentration of lower molecular weight components, reduce the volume of solution and to exchange the buffer to 137 mM acetic acid-triethanolamine-30 mM NaCl, pH 4.5-6.0, the preferred pH being 4.7. Alternatively, the protein solution may simply be filtered through a 0.2 micron filter, pH adjusted to 4.7-6.0 with 1 N HCl or 1 N NaOH, and diluted with water to a conductivity of 4.0 - 7.0 mS/cm. This protein solution was clarified (if necessary) by filtration through a 0.2 micron filter then applied to a 16 x 72 cm WP carboxy-sulfone (weak-strong cation exchanger) (Mallinckrodt Baker # 7587) equilibrated with 137 mM acetic acid-triethanolamine-30 mM NaCl, pH 4.5-6.0, the preferred pH being 4.7 at a flow rate of 100-300 cm/minute. Non-bound components were washed off the column with 3-10 column volumes of equilibration buffer followed by elution of the Protein A with a step of 137 mM acetic acid-triethanolamine-200 mM NaCl, pH 4.5-6.0 or gradient of 0-1.0 M NaCl in the equilibration buffer over a 10 column volume range. Protein A concentration and purity were determined on fractions using an analytical C4 (HIC) reverse phase column and by monitoring absorbance at 214, 275 and 340 nm on a Waters Photodiode Array Detector. Fractions of Protein A were pooled such that the recovery was 85-100% and such that the pool exhibited an A275/A340 ratio of 7.0 or higher. Using diaflow or dialysis, the protein solution pool was exchanged to give 20 mM Tris-Cl, pH 7.8 and to reduce the volume of the solution. This protein solution was applied to a 10.5 X 72 cm column of XWP 500 PolyQuat-35 with a Waters 515 HPLC pump at a flow rate of 5.5 - 8.0 cm/minute. Non-bound components were washed off the column with 3-10 column volumes of fresh equilibration buffer, then the Protein A was eluted from the column with 20 mM Tris-Cl, pH 7.8 adjusted to a conductivity of 18 mS/cm with NaCl. Protein A concentration and purity were determined on fractions using an analytical C₄ (HIC) reverse phase column and by monitoring absorbance at 214, 275 and 340 nm on a Waters Photodiode Array Detector. Fractions of Protein A were pooled such that the recovery is 85-100% and such that the pool exhibits an A275/A340 ratio of 7.0 or higher.

(6) Identity Between Protein A Isolated from Animal-derived and Non-animal-derived Media Protein A was separately purified from *S. aureus* cells grown in animal-derived media or non-animal-derived media using, in sequence, anion exchange then cation exchange in some cases and cation exchange followed by anion exchange in other cases. Each purified protein was then tested for co-migration on SDS gels and non-denaturing gels (Laemmli gels without SDS in either the sample nor in the gel itself). The two separate proteins migrated at the same rate in both systems, respectively. The separate Protein A's were submitted to a commercial lab for amino acid analysis using 6 N HCl, N-terminal sequence analysis and for peptide mapping. For both proteins, the amino acid analysis was comparable. N-terminal analysis of the first six amino acids matched though a small amount of valine appeared in the non-animal-derived Protein A that was not in the first amino acid removed in the animal-derived Protein A. Peptide mapping was carried out on the two proteins by digesting with trypsin and separating the resultant peptides by HPLC. The elution profile of the two were virtually identical, the exception being that two peaks eluted from the non-animal-derived Protein A fragmentation eluting at 61.5 and at 87 minutes was not present In the animal-derived Protein A fragmentation and the peak that eluted at 92 minutes on the animal-derived was much larger on the non-animal-derived. This combination of events could have resulted from incomplete breakdown of a larger peptide in the non-animal Protein A fragments. Otherwise, there was nearly perfect similarity between the two patterns. Each of the two Protein A's were run on isoelectric focusing gels also giving similar patterns, the predominant protein bands having isoelectric points of pH 5.1, 4.9 and 4.7 when compared with standard proteins of horse heart myoglobin, carbonic anhydrase and β-lactoglobulin.

## Claims

1. A non-animal derived Protein A purification process that comprises:
(a) providing a non-animal derived Protein A solution that has been produced by fermenting a secretor strain of *Staphylococcus aureus* in a media containing non-animal derived amino acids or peptides, said media being free of animal products;
(b) filtering said Protein A solution to remove bacterial cells, particulates and low molecular weight molecules (< 10,000 daltons) before loading the Protein A solution onto a first column;
(c) loading the filtered Protein A solution onto the first column;
(d) washing the column with a buffer-salt mixture to rinse off the column undesirable color and contaminating proteins;
(e) eluting the Protein A from the first column to produce a Protein A solution pool;
(f) adjustment of the pH and conductivity of the Protein A solution pool;
(g) loading the Protein A from the Protein A solution pool onto a second column;
(h) washing the second column with a buffer-salt mixture to rinse off the column undesirable color and contaminating proteins; and
(i) eluting the Protein A from the second column to produce a purified Protein A solution;
wherein the first column is a cation exchange column and the second column is a column selected from the group consisting of an anion exchange column, a HIC column and a ceramic hydroxyapatite column, and
wherein the Protein A has a purity of >95% by SDS gel electrophoresis and a pool absorbance ratio at 275nm:340nm of greater than 7.0.

2. A purification process according to claim 1 comprising the additional step: monitoring the absorbance of each fraction of purified Protein A out of the second column at 275 nm and at 340nm to obtain a A275/A340 ratio in order to choose appropriate fractions for pooling such that the pool absorbance ratio is greater than 7.0.

3. A purification process according to claim 1, wherein the second column is a anion exchange column.

4. A purification process according to claim 1, wherein the second column is a HIC column.

5. A purification process according to claim 1, wherein the second column is a ceramic hydroxyapatite column.

6. A purification process according to claim 1 comprising the further step of: drying the purified Protein A solution to a dry product.

7. A purification process according to claim 6, wherein the drying occurs by lyophilization.

## Patentansprüche

1. Reinigungsverfahren für nicht aus Tieren stammendes Protein A, bei dem man
(a) eine Lösung von nicht aus Tieren stammendem Protein A bereitstellt, die durch Fermentation eines Sezerniererstamms von Staphylococcus aureus in einem nicht aus Tieren stammende Aminosäuren oder Peptide enthaltenden Medium hergestellt wurde, wobei das Medium frei von tierischen Produkten ist;
(b)die Protein-A-Lösung zur Abtrennung von Bakterienzellen, partikulärem Material und niedermolekularen Molekülen (< 10000 Dalton) vor dem Beladen einer ersten Säule mit der Protein-A-Lösung filtriert;
(c)die erste Säule mit der filtrierten Protein-A-Lösung belädt;
(d) die Säule mit einem Puffer-Salz-Gemisch wäscht, um unerwünschte Farbe und verunreinigende Proteine von der Säule zu spülen;
(e)das Protein A von der ersten Säule unter Erhalt einer Protein-A-Sammellösung eluiert;
(f)den pH-Wert und die Leitfähigkeit der Protein-A-Sammellösung einstellt;
(g) eine zweite Säule mit dem Protein A aus der Protein-A-Sammellösung belädt;
(h) die zweite Säule mit einem Puffer-Salz-Gemisch wäscht, um unerwünschte Farbe und verunreinigende Proteine von der Säule zu spülen; und
(i)das Protein A von der zweiten Säule unter Erhalt einer gereinigten Protein-A-Lösung eluiert;
wobei es sich bei der ersten Säule um eine Kationenaustauschsäule und bei der zweiten Säule um eine aus der Gruppe bestehend aus einer Anionenaustauschsäule, einer HIC-Säule und einer Hydroxyapatit-Keramiksäule ausgewählte Säule handelt und
wobei das Protein A eine Reinheit von >95% gemäß SDS-Gelelektrophorese und ein 275nm:340nm-Sammellösungsabsorptionsverhältnis von mehr als 7,0 aufweist.

2. Reinigungsverfahren nach Anspruch 1, bei dem man in einem zusätzlichen Schritt jeweils die Absorption der einzelnen Fraktionen von gereinigtem Protein A aus der zweiten Säule bei 275 nm und bei 340 nm erfasst, so dass man ein A275/A340-Verhältnis erhält, um entsprechende Fraktionen für die Sammellösung so zu wählen, dass das Sammellösungsabsorptionsverhältnis mehr als 7,0 beträgt.

3. Reinigungsverfahren nach Anspruch 1, wobei es sich bei der zweiten Säule um eine Anionenaustauschsäule handelt.

4. Reinigungsverfahren nach Anspruch 1, wobei es sich bei der zweiten Säule um eine HIC-Säule handelt.

5. Reinigungsverfahren nach Anspruch 1, wobei es sich bei der zweiten Säule um eine Hydroxyapatit-Keramiksäule handelt.

6. Reinigungsverfahren nach Anspruch 1, bei dem man in einem weiteren Schritt die gereinigte Protein-A-Lösung zu einem Trockenprodukt trocknet.

7. Reinigungsverfahren nach Anspruch 6, wobei das Trocknen mittels Lyophilisation erfolgt.

## Revendications

1. Procédé de purification de protéine A d'origine non animale, qui comprend :
(a) la mise à disposition d'une solution de protéine A d'origine non animale, qui a été produite par fermentation d'une souche sécrétoire de *Staphylococcus aureus* dans un milieu contenant des acides aminés ou des peptides d'origine non animale, ledit milieu étant exempt de produits animaux ;
(b) la filtration de ladite solution de protéine A pour éliminer les cellules bactériennes, les particules et les molécules à faible masse moléculaire (< 10 000 daltons) avant mise en place de la solution de protéine A sur une première colonne ;
(c) mise en place de la solution de protéine A filtrée sur la première colonne ;
(d) lavage de la colonne avec un mélange tampon-sel, pour éliminer par rinçage de la colonne la couleur et les protéines contaminantes indésirables ;
(e) l'élution de la protéine A à partir de la première colonne, pour produire un pool de la solution de protéine A ;
(f) l'ajustement du pH et de la conductivité du pool de la solution de protéine A ;
(g) la mise en place de la protéine A, provenant du pool de la solution de protéine A, sur une deuxième colonne ;
(h) le lavage de la deuxième colonne avec un mélange tampon-sel, pour éliminer par rinçage de la colonne la couleur et les protéines contaminantes indésirables ; et
(i) l'élution de la protéine A à partir de la deuxième colonne pour produire une solution de protéine A purifiée ;
où la première colonne est une colonne d'échange de cations et la deuxième colonne est une colonne choisie dans le groupe consistant en une colonne d'échange d'anions, une colonne de chromatographie par interaction hydrophobe HIC et une colonne d'hydroxyapatite céramique, la protéine A ayant une pureté supérieure à 95 % telle que déterminée par électrophorèse sur gel en présence de SDS, avec un rapport entre absorptions du pool à 275 nm:340 nm supérieur à 7,0.

2. Procédé de purification selon la revendication 1, comprenant l'étape additionnelle de surveillance de l'absorption de chaque fraction de la protéine A purifiée sortant de la deuxième colonne à 275 nm et à 340 nm pour obtenir un rapport A275/A340 dans le but de choisir les fractions appropriées pour le regroupement de telle sorte que le rapport entre absorptions du pool soit supérieur à 7,0.

3. Procédé de purification selon la revendication 1, dans lequel la deuxième colonne est une colonne d'échange d'anions.

4. Procédé de purification selon la revendication 1, dans lequel la deuxième colonne est une colonne HIC.

5. Procédé de purification selon la revendication 1, dans lequel la deuxième colonne est une colonne d'hydroxyapatite céramique.

6. Procédé de purification selon la revendication 1, comprenant l'étape supplémentaire de séchage de la solution de protéine A purifiée, pour donner un produit sec.

7. Procédé de purification selon la revendication 6, dans lequel le séchage a lieu par lyophilisation.
